# EUROPEAN PATENT APPLICATION

(11) **EP 2 258 291 A2**
(43) Date of publication of application: **08.12.2010**
(21) Application number: 10181087.7
(22) Date of filing: 23.07.2003
(51) Int. Cl.: A61B 17/80

(54) **Supporting element for attachment to bone.**

(30) Priority: 23.07.2002 NL 1021137
(62) Divisional of application: 03794345.3
(71) Applicant: Fondel Finance B.V., 3034 KA Rotterdam (NL)
(72) Inventor: McLeod, Allan Gordon, deceased (GB)
(74) Representative: Jansen, Cornelis Marinus

(57) **Abstract**

Supporting element for attachment to bone, comprising means for placement of attachment means and a surface for positioning against said bone, wherein said surface is, at least during use, provided with sponge like biocompatible material, enclosed between said bone and said surface, wherein said sponge like material is provided as granules having an internal porosity and are provided such that at least micro spaces are provided between said granules in which during use bone can grow into and/or from, **characterised in that** said sponge like material comprises titanium, made by a reaction of titanium tetrachloride.

## Description

The invention relates to a supporting element for attachment to bone.

It is known to screw elements onto or into bone, such as plates for bridging a bone fracture. Such plates are normally made of a biocompatible material such as titanium and are screwed into said bone by fixing screws. The plates cannot be screwed down tightly onto said bone without being detrimental to bone growth underneath. Screwing the plate tightly down to said bone would cut off or at least restrict blood vessels in the bone beneath said plate. Bone resorption might occur. This means that mechanical properties of the plate/bone combination would not be optimal, due to the relative looseness of said plate fixation. Moreover, the use of such elements would be limited. For example controlled vibration for inducing and promoting bone growth has to be limited or even prevented and mechanical loading, for example for micro motion control has to be minimised.

The invention is directed to a supporting element, which provides for better connection to bone and better mechanical properties. Previous inventions have tried to use indented patterns on the undersides to avoid this problem.

Object of the present invention is to provide for a supporting element for attachment to bone, which element can be attached relatively tightly without cutting off restricting blood vessels, nerves or the like.

Further object of the present invention is to provide for a supporting element, which can be attached to bone, which element during use initiates and/or promotes bone growth.

A still further object of the present invention is to provide for a supporting element which can be mechanically loaded almost directly after attaching said element to bone.

A still further object of the present invention is to provide for a method for fixating a supporting element on bone.

These and further objects of the present invention are achieved by a supporting element characterised by the feature of claim 1.

By providing a biocompatible, sponge like material on a surface of said element that is to be positioned against bone onto which it is to be attached, the element can be attached relatively tightly, whereas said sponge like material provides for space into which bone could grow. Moreover, the sponge like material would prevent cutting off or restricting blood vessels underneath said element. Bone resorption will thus be prevented, at least substantially.

Since an element according to the present invention can be attached relatively tightly onto bone, it can be loaded mechanically, even directly after fixation. The element can for example be vibrated for inducing or promoting bone growth or a known mechanical device can be attached to said element for inducing or controlling micro motion of said element.

Preferably said sponge like material is provided at least partly as granules having an internal porosity. The granules are preferably provided by using material which has been produced by a reaction with titanium tetrachloride, crushed maintaining internal micro porosity. Said granules can easily be positioned on a surface of said element, providing for a favourable pressure distribution, whereas they provide for space into and/or from which bone can grow. The granules can, if desired, be mixed with for example bone grind or other bone growth inducing and/or promoting means such as chemicals, blood or the like.

The at least one surface of the element which has been or is to be provided with said bio compatible sponge like material can be provided with at least one hollow part, for example by providing a standing surrounding wall which is, when the element is screwed down, at least almost engaging said bone, keeping at least part of said surface at a distance from said bone, said sponge like material being positioned in between. This leads to easy enclosure of said material. Said surface may be bent, at least in one direction, for a better fit on for example so called long bones. It will however be clear that any shape, size or form may be suitable.

The present invention further relates to tape, provided on at least one side with sponge-like biocompatible material such as titanium based material. This tape is especially suitable when said titanium based material is made by a reaction of titanium tetrachloride, the tape being relatively flexible. Such tape, which is inventive in itself, is especially useful in combination with for example screw down elements according to the invention. A strand of tape can be positioned on the element and/or on the surface of said element before positioning of said element. The amount of tape and possibly the kind of tape can then be chosen for example depending on the patient and the type of element. The amount and type of material on the tape surface, as well as for example the distribution can then for example be varied. The tape can also be used individually, that is apart from said element, for example for wrapping bone, or could be crumpled up for use as filler.

The present invention furthermore relates to a set of a supporting element such as a screw down element and granules of a sponge-like, biocompatible material having an internal porosity, means being provided for enclosing said granular material between said element and bone. The granular material may to that end be provided with or mixed with adhesive means such as blood, biocompatible adhesives or the like, or may be provided on said tape or similar means. Also mechanical means may be used preferably (semi) permeable to bone growth, blood or the like

In a preferred embodiment a set according to the present invention is **characterized in that** tape according to the present invention is provided on at least one surface of the supporting element or at least be attached thereto for at least providing for part of the attachment of said element to bone.

Such set has the advantage that the element can be attached to bone at least provisionally using said tape, provided that said tape and/or said granules are provided with adhesive means on both sides. This enables easy positioning of said supporting element and screwing down said element, if necessary. The plate can also be attached to said bone by wrapping said tape around the element and said bone. Obviously, the plate can also be provided with adhesive means itself.

The invention further relates to use of biocompatible granules, according to claim 15 and to preparation of an element to be attached to bone, according to claim 16.

The invention further relates to a method for attachment of an element onto bone, characterized by the features of claim 17.

In a method according to claim 17 an element can easily be attached to bone, especially on for example a relatively smooth, flat or curved outer surface thereof, preventing cutting off or restriction of blood vessels, nerves or the like in said bone. The granules further provide for the advantage that they may promote bone growth, or at least prevent resorbtion.

In the further claims further advantages embodiments of the present invention are described, which are incorporated in this introduction.

For a better understanding of the present invention by way of examples, embodiments are shown and described of a supporting element, set, tape, use, preparation and method according to the present invention, referring to the drawings. These show:
Figure 1 schematically in perspective view a supporting element according to the present invention, without granules, seen from a front side;
figure 2 the element according to figure 1, from the back side;
figure 3 the element of figures 1 and 2, from the front side, partially filled with granules;
figure 4 and 4A in cross-section an element according to the present invention, screwed to bone;
figure 5 an element according to the present invention, in a first alternative embodiment, screwed down over a broken bone;
figure 6 in side view a roll of tape according to the present invention;
figure 7 the tape of figure 6, cut out and in frontal view;
figure 8 in perspective view tape according to figures 6 and 7, partly crumpled; and
figure 9 a supporting element according to the present invention, positioned on bone by tie means .

In this application the same or corresponding parts have the same or corresponding reference signs. The embodiments are only shown by way of example and should not be understood as limiting the invention in any way. In this description the term "granules" is used for relatively small particles of a biocompatible material such as metal, preferably being sponge-like having at least micro porosity, which granules may be broken or ground from larger particles and which are preferably made by a reaction of titanium tetrachloride. The granules are preferably crushed in such a way as to maintain their internal micro porosity. Granules of different sizes and different materials may be used separately or in combination.

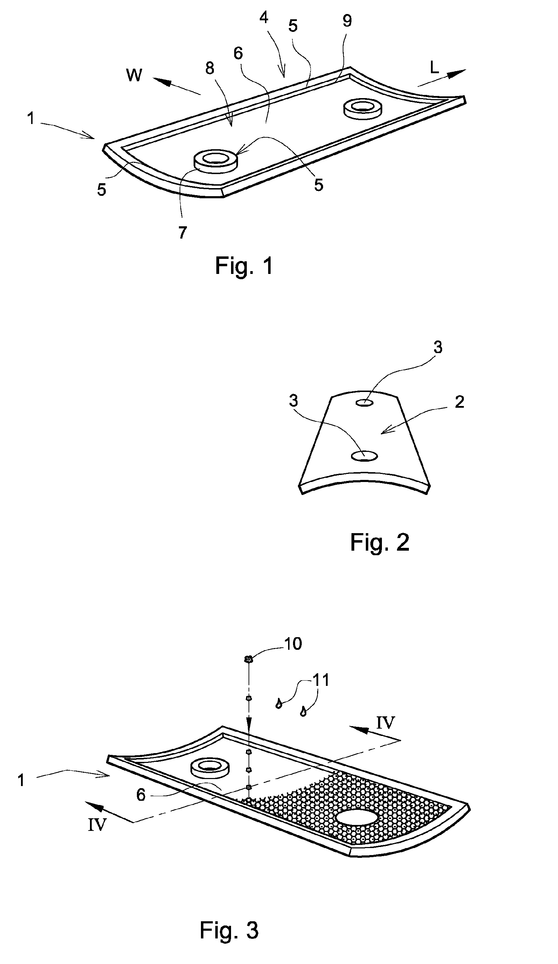

Figure 1-3 show a supporting element in a first advantageous embodiment, which supporting element can be referred to as a screw down plate 1. The plate 1 is made of a biocompatible material such as but not limited to titanium, titanium alloy or a biocompatible or biodegradable plastic. The plate 1 has a bend configuration, such that in longitudinal direction L it is substantially straight and in widthward direction W it is curved, basically forming a ring sector. The plate 1 is on the convex outer side 2 substantially smooth and is provided with two through bores 3 extending through said plate. On the opposite bottom side 4, which during use is directed towards bone onto which the plate 1 is to be screwed, an upstanding wall 5 is provided extending around the outer edge of said plate and enclosing an inner surface 6 which is concave. The through bores 3 are on the bottom side 4 of the plate 1 also surrounded by a wall 7. Thus the plate 1 is provided with at least 1 hollow or indentation 8, formed by the lower surface 6 and the inner edge 9 of the wall 5 and the wall 7.

As can be seen from figure 3, said indentation or hollow 8 may be completely or partly filled with a biocompatible granular material, further to be referred to as granules 10 as discussed here above. The granules 10 are position in said hollow or indentation 8 by using for example adhesive means 11, such as blood, biocompatible adhesives, gelatine or the like, such that the granules 10 are at least provisionally adhered to each other and/or to the plate 1, especially to said lower surface 6. It will be clear that the granules 10 may also be provided in a different manner, for example by enclosing granules in a pouch, netting or the like, such that they can be positioned on at least part of at least one surface of said plate, especially on the bottom surface 6. Especially in such embodiment the bottom surface 6 may also be completely or substantially flat, that is without an upstanding wall 5.

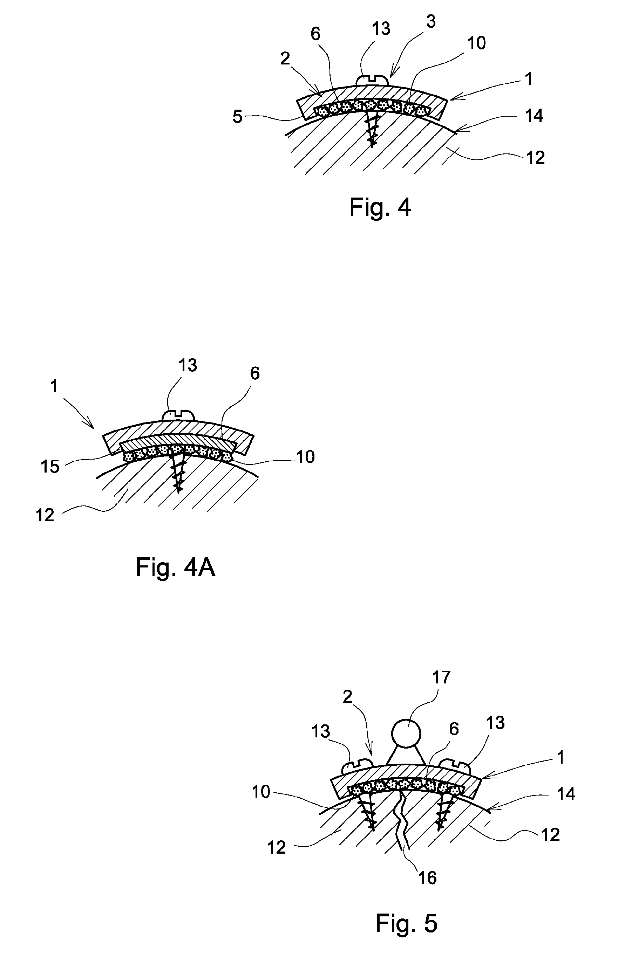

In figure 4 in cross-section, along the line IV-IV in figure 3 an element 1 is shown screwed down onto bone 12 by means of screws 13 extending through said through bores 3 and screwed into the bone 12. The granules 12 are hereby enclosed between the outer surface 14 of the bone 12 and the inner surface 6 of the element 1. The screws 13 can be screwed down relatively firmly, squeezing the granules 10 between said two surfaces 6, 14, without the risk of cutting off nerves, blood vessels or the like in the bone 12, since the element 1 is screwed down by the screws 13 and rests on the surface 14, mainly through the granules 10.

In figure 4A an alternative embodiment of an element 1 is shown, in the same cross-section as in figure 4, in which the granules are adhered to the element 1, especially to said inner surface 6 by means of a layer 15 of adhering material, such as two-sided adhesive tape or a layer 15 of biocompatible glue. It will be clear that this layer 15 may be made of any material having adhering properties, including gum-like materials. In this embodiment the element 1 is once again screwed down into bone 12 using screws 13.

In figure 5 a further alternative embodiment of an element 1 according to the present invention is screwed down onto bone 12 using screws 13, enclosing granules 10 between an inner surface 6 and the outer surface 14 of the bone 12. In this embodiment the element 1 extends over a braking surface 16 in bone 12 and is on the outer surface 2 provided by a connecting element 17, for example a knob-like element to which for example connecting means can be connected for an outer harness, vibrating means or the like for mechanically loading said element 1, for example for inducing or controlling micro motion of said element. Such means are known from the prior art.

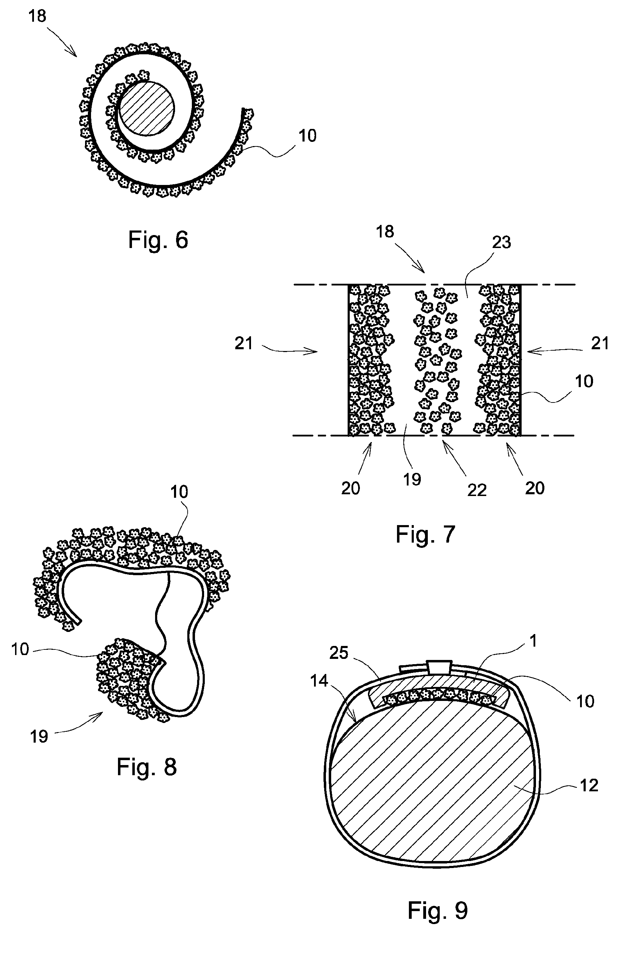

Figures 6-8 show tape 18 according to the present invention, provided on at least one outer surface 19 with granular material 10 as discussed above. This granular material 10 can be positioned on said outer surface 19, completely filling said surface, as shown in figure 8, the granules 10 being substantially of similar size and compactly provided on said surface. The granules 10 may also be provided on said surface in specific patterns, for example as shown in figure 7, in which two areas 20 of relatively densely packed granules are provided along the outer edges 21 of said tape 18, whereas an area 22 is provided along the middle of said surface 19 with less densely packed granules. Said areas 20, 22 may be separated by areas 23 without any granules. Figure 7 shows only one example of such pattern. Any pattern is obviously possible with said granules. Such tape may be made by providing an adhesive on said surface 19, onto which the granules are stuck. The granules themselves may also be provided with an adhesive material, such as blood, gelatine, biocompatible glue or the like. In such embodiment the tape 18 may be stuck onto any surface, such as bone or the surface of an element 1 according to the present invention by means of said adhesive means.

Tape 18 according to figures 6-8 may be used as a layer combining the layer 15 and the granules 10 as shown in figure 4A, enclosed between an element 1 according to the present invention and bone 12. However, said tape 18 may also be folded, kneaded, crumpled or the like in order to provide for bone filler or the like. In an alternative embodiment the tape 18 may be wrapped around the outer surface 2 of an element 1 and bone 12, wherein the tape 18 can be adhered to said element 1, said bone 12 and/or to itself.

In figure 9 an alternative embodiment of a set according to the present invention is shown in which an element 1 with granules 10, such as shown in figure 3, is attached to an outer surface 14 of bone 12 by means of tie means 25, for example biocompatible plastic tie straps, elastic bands or the like. As tie means 25 also tape 18 may be used.

By using a set according to the present invention of for example a supporting element 1, tape 18 and/or attachment means such as screws, said tape and/or tie straps or the like, a supporting element 1 according to the present invention can be positioned on a surface 14 of bone such as skeleton parts of a human body, for example extremities, which can be fixed relatively tightly onto said bone, preventing cutting off of blood circulation, nerves or the like and preventing bone resorbtion. The supporting element can be loaded with outside forces almost immediately after attachment to said bone. The granular material 10 will allow and possibly even promote bone growth into said granules, for obtaining an even better attachment. The granules can be mixed with for example bone material, especially of the patient, which may induce bone growth into and from said granular material, even further enhancing the attachment of said element 1.

The present invention is by no means limited to the embodiments described and shown in the drawings. Many further elaborations and embodiments of the present invention are feasible within the scope of the invention defined by the claims or in the description.

Tape 18 according to the present invention can for example be provided on both sides with granular material, which may be similar material or completely different materials. A supporting element 1 according to the present invention may have any shape, size or configuration, suitable for specific bone structures, such as long bones, short bones, pelvis, shoulders and the like. Tape 18 according to the present invention is especially suitable for use with an element 1 according to the present invention, but may also be used separately. The granules may be at least partly replaced by other biocompatible material distributing attachment forces in a similar way, such as rough surfaced mats or the like. Instead of screws 13 other attachment means may be used, such as nuts and bolts extending through said bone, nails or the like.

## Claims

1. Supporting element for attachment to bone, comprising means for placement of attachment means and a surface for positioning against said bone, wherein said surface is, at least during use, provided with sponge like biocompatible material, enclosed between said bone and said surface, wherein said sponge like material is provided as granules having an internal porosity and are provided such that at least micro spaces are provided between said granules in which during use bone can grow into and/or from, **characterised in that** said sponge like material comprises titanium, made by a reaction of titanium tetrachloride.

2. Supporting element according to claim 1, wherein said surface is provided with at least one hollow in which said material is provided.

3. Supporting element according to any one of the previous claims, wherein said material is adhered to said at least one surface.

4. Supporting element according to any one of the previous claims, wherein said element is or has at least one partly plate shaped element having said at least one surface on which said sponge like material is provided.

5. Supporting element according to any one of the previous claims, wherein said surface is bent in at least one direction.

6. Supporting element according to any one of the previous claims, wherein bone growth promoting means are provided on and/or in said sponge like material and/or on said surface, especially bone growth inducing or promoting chemicals.

7. Set of a supporting element and granules of a sponge like, bio compatible material having an internal porosity, said supporting element having a surface which can be attached to, especially screwed down onto bone, wherein means are provided for positioning said granules between said bone and said surface **characterised in that** said granules are made with titanium, by a reaction of titanium tetrachloride.

8. Tape comprising on at least one side sponge-like biocompatible material which is titanium based material, especially made by a reaction of titanium tetrachloride, which tape is relatively flexible.

9. Tape according to claim 8, which tape is at least partly adhesive, such that it can be stuck to an element to be fixed to a bone and/or to bone and/or to itself.

10. Tape according to claim 8 or 9, sufficiently flexible to be folded or crumpled into any shape for use as filler.

11. Supporting element according to any one of claims 1-7, wherein tape according to any one of claims 8-10 is provided on a surface thereof.

12. Use of biocompatible granules having an internal porous structure in screwing or adhering an element onto bone, **characterised in that** said granules are made with titanium, by a reaction of titanium tetrachloride..

13. Method for the preparation of an element to be attached to a bone, wherein biocompatible granules having an internal porous structure are positioned on a surface to be positioned facing said bone, such that these granules will be enclosed between said surface and said bone, wherein said granules are made with titanium, by a reaction of titanium tetrachloride.

14. Method for attaching an element onto bone, wherein biocompatible granules having an internal porous structure are positioned between a surface of said element and said bone, wherein said element is attached tightly against said bone and bone growth into and between said granules is promoted, wherein said granules are made with titanium, by a reaction of titanium tetrachloride.

15. Method according to claim 13 or 14, wherein the granules are brought into contact with the surface of the element.

16. Biocompatible granules having an internal porous structure for use in screwing or adhering an element onto bone, wherein said granules are made with titanium, by a reaction of titanium tetrachloride.
